# EUROPÄISCHE PATENTANMELDUNG

(11) **EP 1 062 976 A2**
(43) Veröffentlichungstag der Anmeldung: **27.12.2000**
(21) Anmeldenummer: 00250211.0
(22) Anmeldetag: 26.06.2000
(51) Int. Cl.: A61N 1/365

(54) **Medizinisches Therapiesystem**

(30) Priorität: 26.06.1999 DE 19930533
(71) Anmelder: BIOTRONIK Mess- und Therapiegeräte GmbH & Co Ingenieurbüro Berlin, 12359 Berlin (DE)
(72) Erfinder: Schaldach, Max, 91054 Erlangen (DE); Ameling, Walter, 52074 Aachen (DE)
(74) Vertreter: Eisenführ, Speiser & Partner

(57) **Zusammenfassung**

Medizinisches Therapiesystem, vorzugsweise zur Elektrostimulation oder sonstigen Behandlung des Herzens mit elektrischen Impulsen mit zwischen eingangsseitigen Sensormitteln und ausgangsseitigen Therapie-Applikatormitteln eingeschalteten Verknüpfungs- und/oder Bearbeitungsstufen für Signale , wobei aus den Eingangsignalen eine Signalgröße durch die Verknüpfungs-/Bearbeitungsstufen und/oder in einem aus mehreren Verknüpfungs-/Bearbeitungsstufe bestehenden Verknüpfungs-/Bearbeitungsmodul aus den Eingangsignalen mindestens ein Indikatorsignal ermittelt wird, das ein Maß für ein wahrscheinlich bevorstehendes therapiepflichtiges Ereignis oder den Therapieerfolg bildet.

## Beschreibung

Die Erfindung betrifft ein - insbsondere implantierbares - medizinisches Therapiesystem, vorzugsweise ein System zur Elektrostimulation oder sonstigen Behandlung des Herzens mit elektrischen Impulsen.

Derartige Systeme sind beispielsweise als implantierbare Herzschrittmacher, Defibrillatoren oder entsprechende Geräte zur Beseitigung von Arrhythmiezuständen des Herzens oder zur Elektrostimulationsbehandlung von anderen Teilen des menschlichen Körpers bekannt.

Die entsprechenden Geräte erzeugen elektrische Impulse oder sonstige auf den menschlichen Körper einwirkende Signale oder Therapiewirkungen in Abhängigkeit von Sensorsignalen, die sie aus dem Körper aufnehmen. Es ist aber auch eine Steuerung von extern möglich.

Die bisherigen derartigen Geräte weisen den Nachteil auf, daß sie meist nur solche Informationen verarbeiten konnten, welche in unmittelbarer Beziehung zur Therapie standen, d.h. sowohl zeitlich als auch was die Verarbeitung der Signale betrifft, der unmittelbaren Therapie sehr nahe standen und somit in der Ursache-Wirkungs-Verknüpfung verhältnismäßig leicht zu beherrschen waren. Für das Beispiel eines Herzschrittmachers bedeutet dies, daß zur Steuerung der Stimulation solche Signale aus dem Herzen aufgenommenwurden, welche mit dem Behandlungserfolg unmittelbar korreliert sind. Das einfachste diesbezügliche Beispiel bildet ein Demand-Schrittmacher, welcher immer dann Herzschläge künstlich durch Stimulation hervorruft, wenn natürliche Herzschläge ausfallen. Ersichtlicherweise reichte hier ein einfacher durch einen natürlichen Herzschlag zurücksetzbarer Zeitgeber aus, um dieses Geräte in seiner Grundfunktion zu realisieren.

Bei komplexeren arrhythmischen Zuständen sind aber die Verknüpfungsbeziehungen von durch Sensoren im Körper aufzunehmenden Signalen weitaus komplizierter.

Der Erfindung liegt daher die Aufgabe zugrunde, auch Informationen aus dem Körper zu Therapiezwecken nutzbar zu machen, welche einerseits relativ verdeckt im Signalgeschehen des Körpers erkennbar sind und andererseits auch nur einen entfernteren zeitlichen Bezug zu dem aktuellen Signalgeschehen haben.

Diese Aufgabe wird durch das erfindungsgemäße System gelöst, welches es durch die zeitrelative Verknüpfung von Systemaussagen ermöglicht Informationen zu verknüpfen, welche sehr weit zeitlich zurück und sehr verdeckt im Signalgeschehen verborgen sind. Hierbei können insbesondere auch Aussagen gewonnen und ausgewertet werden, welche erst für das zukünftige Systemverhalten eine Bedeutung haben.

Erstmals können somit in die Steuerung von automatisch arbeitenden Therapiegeräten klinische Indikatoren, wie Risikofaktoren, Lebensqualitätsindikatoren, Risikofaktoren und klinische Folgeindikatoren oder Leistungsfaktoren im System gewonnen und in die Berechnungen mit einbezogen werden. Dies gelingt durch mehrstufige Verarbeitung, welche zielbezogen auch verborgen liegende Informationen in den aus dem Körper des Patienten aufgenommenen Signalen aufspüren kann.

Da auch Therapiesysteme wegen der im Körper vorhandenen Zeitkonstanten eine Therapie möglichst frühzeitig mit zeitlichem "Vorhalt" einleiten sollten, kann damit bereits einem möglicherweise in der Zukunft auftretenden Ereignis so entgegengewirkt werden, daß das Ereignis gar nicht erst eintritt. Hierbei kommt dem hier vorgestellten System zugute, daß es nicht an die Lösungen herkömmlicher Regelungstechnik gebunden ist, sondern, daß es Aussagen aller Art, die sich in einem komplexen Signal-Daten-Set ausdrücken lassen miteinander verknüpft. Da die Verknüpfung mehrstufig ist konvergiert die Signalaussage, wegen der somit erfolgenden Informationskonzentration, relativ schnell, so daß den gewonnenen Therapiemaßnahmen eine hohe Sicherheit innewohnt. Durch diese Informationskonzentration wird jeweils in einem Modul unter einer verbalen Bezeichnung die relativ höchstwertige Aussage mit einem Zeitbezug gehalten. Die betreffenden Signal-Daten-Sets können bzw. müssen gegebenenfalls noch weitere Angaben enthalten, welche ihren Gültigkeitsbereich betreffen. Diese bilden Aussageverknüpfungen, welche eine Therapiebedingung oder Betriebsart beinhalten, für welche die betreffende Aussage jeweils nur eingeschränkt gültig ist. Derartige die Aussagegültigkeit beschränkende Vektoren können Zusatzbedingungen sein, welche auf andere Signalzustandssets oder auf andere Zeitpunkte bzw. periodische Zeitsequenzen hinweisen und diese damit logisch "verklammern". Auf diese Weise können die gefundenen "Kontextinformationen" bei der nachfolgenden Verarbeitung im Kontext verweisen (gültig, weil Bedingung xyz erfüllt). Auf diese Weise kann bei der späteren Verarbeitung auf die Ermittlung der Zusammenhangsaussagen durch Verarbeitung/Verknüpfung verzichtet werden, so daß sich die Verarbeitung beschleunigt. Dies gilt für redundante Kontextinformationen, welche also nicht zwingend vorhanden sein müssen. Es gibt aber auch nicht redundante Kontextinformationen wie Zeitverweise auf Bezugszeitperioden, welche insbesondere beim Herzgeschehen ein eigenes Zeitbezugssystem bilden. Die Bezugszeitperioden für Herzschläge werden im Echtzeitsystem gesetzt, halten aber einen Zeitvektor für das nächste zu erwartende periodische Ereignis. Ändert sich dann die Herzrate, so wird dieser Vektor durch die entsprechende Verknüpfungs/Verarbeitungseinheit verändert mit der Folge, daß sich auch die entsprechenden Bezugszeiten ändern.

Besonders vorteilhaft bei dem erfindungsgemäßen System ist der Umstand, daß sich sowohl die die Verarbeitungs- und Verknüpfungseinheiten bestimmenden Verarbeitungs- und Verknüpfungsalgorithmen als auch die als Verknüpfungsaussagen gewonnenen Signal-Daten-Sets über mehrere Systeme austauschen lassen. Es können Simulationen außerhalb des individuellen Systems ausgeführt werden. Aufwendige Verarbeitungsvorgänge können über die Kommunikationsmittel "ausgelagert" oder besondere Algorithmen (Verbesserungen, Speziallösungen) als "Plug-Ins" eingelagert werden. Die einzelnen Verarbeitungs und Verknüpfungseinheiten sind in ihrem Aussageverhalten durch die benutzten verbalen Kontexte, mit denen die Signalgrößen belegt sind, ohne besondere Programmierkenntnisse zugänglich, so daß die von medizinisch gebildeten Fachleuten selbst definiert werden können. Auf diese Weise konvergiert die Hochwertigkeit der gewonnenen Aussagen nicht nur durch mehrstufige intelligente Verknüpfung innerhalb eines Systems und die über die Zeitdauer gewonnen Erfahrungen, sondern die Aussagesicherheit gewinnt zusätzlich durch die Erfahrung, die sich aus der Querverknüpfung der Erfahrungen aller gleichwertigen Systeme gewinnen läßt. Auf diese Weise können unscheinbar im Signalgeschehen auftretende Ereignisse mikroskopisch herausgefiltert und zu hochwertigen Informationen mit erheblichem Entscheidungsgehalt "verstärkt" werden.

Die notwendigen Therapieaktionen werden aufgrund der entsprechenden Aussageverknüpfungen ermittelt und mit ihren entsprechenden Bezugszeitpunkten definiert. Sollten sich Nebenbedingungen oder Voraussetzungen ändern können die entsprechenden Therapiemaßnahmen entfallen, wenn eine Gegenmaßnahme eingeleitet wird. Wird also durch Prädiktion eine Arrhythmie oder Fibrillation erwartet, kann eine mehrstufige Reaktion eingeleitet werden, wenn ein Prädiktor ein solches Ereignis in der Zukunft mit einer gewissen Wahrscheinlichkeit anzeigt. Durch den für einen Zeitpunkt in der Zukunft unter der entsprechenden verbalen Bezeichnung angezeigten zu therapierenden Ereignis wird eine zunächst "sanfte" Gegenmaßnahme ausgelöst. Anschließend wird das Fortbestehen des Prädiktors durch Wiederholung der Abfrage (Erneutes Ausführen der entsprechenden Verknüpfungskette mit den dann bestehenden aktuellen Signalzuständen wiederholt. Besteht der Prädiktor fort, wird aufgrund der dann bestehenden Signalzuständen eine "härtere" Gegenaßnahme ausgeführt. Führt auch sie nicht zum Erfolg wird als letzte Gegenmaßnahme bei einer tatsächlich eingetretenen Fibrillation ein Defibriallationsschock ausgelöst. Es ist ersichtlich, daß bei den Signal-Daten-Sets die zukünftigen Ereignissen zugeordneten Prädiktoren variabel sind, während klinische Indikatoren, die sich auf die Gegenwart beziehen in ihrer Gültigkeit "eingefroren" werden. Das System ermittelt damit das Therapieverhalten durch die verknüpften Baugruppen kontinuierlich und trifft eine Vorausschau für zukünftige Ereignisse, denen die aktuelle Therapie angepaßt wird. So wird der Herzzyklus zeitlich durch das Setzen entsprechender Datensätze für die jeweiligen Bezugszeitpunkte der Echtzeit und Berücksichtigung des Phasenverhaltens im Herzrhythmus "vorgeplant" und bis zum tatsächlichen Eintreten der zukünftigen Ereignisse in der Planung aktualisiert. Erst das tatsächliche Eintreten beispielsweise eines Herzschlags führt zu dessen tatsächlicher Registrierung als "eingefrorenes" Ereignis-Signal-Daten-Set. Die Abweichung zwischen vorgeplantem und tatsächlich eingetretenem Ereignis führt über eine entsprechende Verknüpfungs-/Verarbeitungseinheit ebenfalls zu einer Aussage, die die Nachstellung eines entsprechenden Systemparameters bewirkt, der ebenfalls als Signal-Daten-Set festgehalten ist.

Neben dieser vorgenannten echtzeitbezogenen Verarbeitung können "Service-Bearbeitungen" wie eine Signalkompression, eine "Verdichtung" von Ereignissen die weiter in der Vergangenheit liegen, zu einer statistischen Zusammenfasssung. Auf diese Weise lassen sich die Daten um so mehr komprimieren je weiter sie in der Vergangenheit liegen. Es läßt sich aber auch ein unkomprimierter Datensatz vor dessen Komprimierung durch Telemetrie "auslagern", so daß interaktiv für eine übergreifende Bearbeitung noch auf den Gesamtdatensatz zurückgegriffen werden kann. Als weitere "Service-Funktionen" können Stabilitätsüberprüfungen und Funktions- und Plausibilitätskontrollen zu Zeiten durchgeführt werden, zu denen kein besonders großer Bearbeitungsbedarf besteht. Dazu gehört auch die Kontrolle des Therapieerfolgs und eine daraus resultierende Selbstlernfunktion. Es ist ersichtlich, daß sich derartige Erfahrungen durch Telemetrie und Kommunikation mit entsprechenden Zentren auch für andere gleichartige Therapiegeräte nutzbar machen lassen.

Zu den Einzelheiten wird auf die beigefügten Ansprüche verwiesen.

Vorteilhafte Weiterbildungert der Erfindung sind im übrigen in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Gesamtübersicht des erfindungsgemäßen Herzstimulationssystems als Blockschaltbild,
- Figur 2: ein Ausführungsbeispiel des Verarbeitungsteils als Detail zu Figur 1 in einfacher Form,
- Figur 3: ein Ausführungsbeispiel des Verarbeitungsteils als Detail zu Figur 1 in komplexerer Form,
- Figur 4: eine Verknüpfungs-/Bearbeitungsstufe als des Detail des Verarbeitungsteils,
- Figur 5: die Darstellung einer Anzahl funktionsbezogener verknüpfungs-/Bearbeitungsmodule, welche jeweils eine Anzahl von Verknüpfungs-/Bearbeitungsstufen enthalten.
- Figur 6: die Darstellung einses Vorhersagemodells nach dem Prinzip der Erfindung sowie
- 13 Darstellungen: im Anhang zur Funktionalität verschiedener nach dem erfindungsgemäßen Konzept realisierter Systeme oder Systemteile.

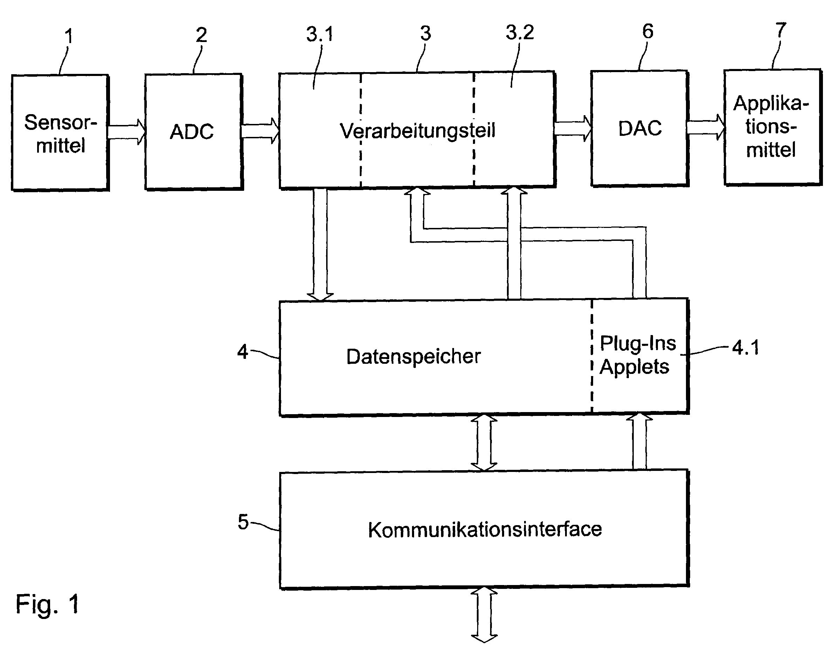

Aus der in Figur 1 dargestellten Gesamtübersicht des erfindungsgemäßen Therapiesystems ist dessen grundsätzliche Aufbau ersichtlich. Hier sind die physikalischen Grundeinheiten beschrieben, welche zur Realisierung verwendet werden. Die in den nachfolgend dargestellten Baugruppen beschriebenen Elemente sind dagegen mehr funktionsbezogen und geben die Arbeitsweise im Detail wieder. Bei dem dargestellten Therapiesystem handelt es sich um ein implantierbares System zur Beseitigung von arrhythmischen Herzzuständen durch Elektrostimulation bzw. Reizschockabgabe, wie als Herzschrittmacher oder ICD (Implantierbarer Cardioverter Defibrillator) bekannt ist.

Die Eingangsschnittstelle zum Patienten wird durch eine Anzahl von Sensoren gebildet, welche in der Zeichnung zu Sensormitteln 1 zusammengefaßt sind. Die einzelnen Sensoren können dabei physikalische oder chemische Wandler sein, welche Signale am Körper abgreifen und in zeitabhängige elektrische Signale umformen. Die Wandler bilden typischerweise Elektroden oder sonstige Signalgeber, welche eine Aussage über den Patientenzustand ermöglichen. Die elektrischen Signale werden Analog-Digital-Wandler-Mitteln 2 zugeführt. Deren digitalisierten Ausgangs-Signale gelangen zu einem Verarbeitungsteil 3, der durch einen Datenprozessor gebildet wird, welcher mit einem Datenspeicher 4 in Wechselwirkung steht. Der Verarbeitungsteil 3 greift auf einen Datenspeicher 4 zu, welcher sowohl die Patientenzustandsdaten, die den Patienten charakterisieren, als auch Systemzustands- und Systemkonfigurationsdaten, welche das dem Patienten zugeordnete System beschreiben, enthält. Der Verarbeitungsteil ist prioritätsgesteuert, führt also die anstehenden Signalverarbeitungsvorgänge nach Dringlichkeit durch, wobei allerdings die Verarbeitung in Bezug auf einen Eingangsteil 3.1 und einen Ausgangsteil 3.2 echtzeitbezogen verläuft, da die entsprechenden Eingangs- und Ausgangssignale zeitgetreu aufgenommen bzw. zur Verfügung gestellt werden müssen. Die übrige Verarbeitung kann hingegen zeitentkoppelt und damit entsprechend der unterschiedlichen Dringlichkeit erfolgen, wobei bestimmte Verarbeitungsaufgaben komplexerer Natur zeitversetzt oder extern vernetzt bearbeitet werden können, wie es weiter unten näher beschrieben ist.

Der Ausgangsteil 3.2 des Verarbeitungsteils 3 steht mit Digital-Analog-Wandler-Mitteln 6 in Verbindung, welche die Schnittstelle zu Therapieapplikatormitteln 7 bilden, die auf den Patienten einwirken. Hierbei handelt es sich um die eigentliche Therapieoberfläche in Form von Elektroden oder chemischen Dosiergeräten.

Systemzustands- und Systemkonfigurationsdaten können einschließlich der zugehörigen Verknüpfungs- und Verarbeitungsalgorithmen von extern in den Verarbeitungsteil "nachgeladen" werden. Derartige zusätzliche Sysemkonfigurationsdaten werden in dem Plug-In-Teil 4.1 des Datenspeichers 4 festgehalten. Der Inhalt des Datenspeichers kann über das Kommunikationsinterface 5 nach extern ausgelesen oder von dort geändert werden. Auf diese Weise ist sowohl eine Auslagerung der Verarbeitung von Daten möglich als auch die Zufuhr von externen Systemzustands- und Systemkonfigurationsdaten sowie von Kollektiven von Patientendaten und verallgemeinerten Patientenzustandsgrößen. Die prioritätsbezogene Verarbeitung erlaubt es, im Rahmen einer Verarbeitung mit niedriger Priorität bei geringem übrigen Verarbeitungsanfall auch vorsorgliche Berechnungen anzustellen, welche möglicherweise benötigt werden, im übrigen aber auch verworfen werden können.

Die Speicherung der Daten erfolgt in sogenannten Signal-Daten-Sets, als Gruppen von einander zugeordneten Informationen. Diese Signal-Daten-Sets weisen Zeitmarken- und Kontextsignale auf, wie es weiter unten näher beschrieben werden wird.

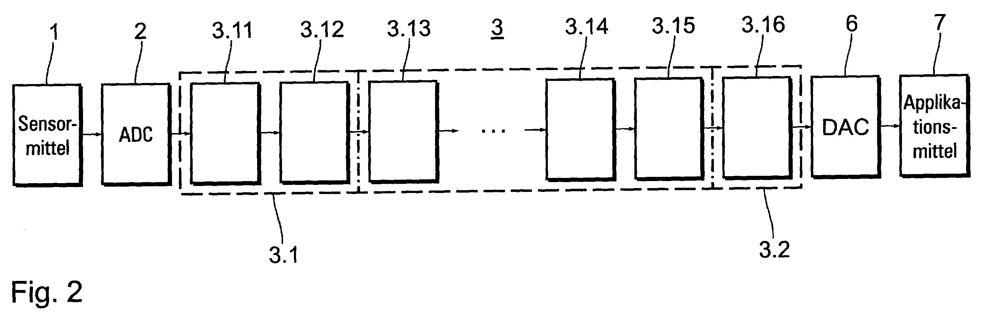

In Figur 2 ist der prinzipielle Verlauf einer einfachen Signalverarbeitung bei unverknüpfter Verarbeitung von den den Sensormitteln 1 zu den Applikatormitteln 7 näher dargestellt. Die Anordnung der Analog-Digital-Wandlermittel 2 und der Digital-Analog-Wandlermittel 6 entspricht derjenigen von Figur 1. Der Verarbeitungsteil 3 von Figur 1 ist hier aber in Form von hintereinandergeschalteten Blöcken wiedergegeben.

Dabei wird mindestens ein digitalisiertes Sensorausgangssignal als Eingangssignal nacheinander zwei ersten (vorgeschalteten und nachgeschalteten) Verarbeitungs-/Verknüpfungsstufen 3.11 und 3.12 zugeführt. Dasverarbeitete Sensorsignal bildet das Ausgangssignal der nachgeschalteten ersten Verarbeitungs-/Verknüpfungsstufen 3.12. Die beiden ersten Verarbeitungs-/Verknüpfungsstufen 3.11 und 3.12 bilden gemeinsam eine Stufe zur Vorverarbeitung der Sensorsignale. Diese Vorverarbeitung dient in der ersten Stufe der Geometriebewertung. Dadurch ist beispielsweise bei Herzstimulatorsystemen eine objektivierte Verarbeitung von Signalen möglich, welche aus unterschiedlichen Elektrodenkonfigurationen stammen. Ausgangssignale von Zusammenschaltungen von mehreren Elektroden werden in dieser Stufe gegebenenfalls verknüpft. In der nachgeschalteten der nachgeschalteten ersten Verarbeitungs-/Verknüpfungsstufen 3.12. erfolgt gegebenenfalls eine Zeit-Frequenzbewertung des Sensorsignals oder eine sonstige Signalbewertung. Diese Signalbewertung ist zunächst nur auf die aktuellen Sensorsignale selbst bezogen und entspricht einer Kurzzeitbewertung oder Filterung um feste Stör- oder Laufzeiteinflüsse auszugleichen, die nicht von anderen Signalen oder Systemzuständen abhängig sind. Dieser Ausgleich umfaßt aber auch die Sensorsignale untereinander. Weiter unten ist beschrieben, wie diese Bearbeitung aufgrund der weiteren Merkmale des beschriebenen Systems auch verknüpft mit anderen Signalen und zeitlich iterativ vorgenommen werden kann. Dieser Verarbeitungsteil wurde in der Baugruppe 3 als Eingangsteil 3.1 bezeichnet.

Das verarbeitete Sensorsignal als Ausgangssignal der ersten Verarbeitungs-/Verknüpfungsstufe 3.11/3.12 bildet das Eingangssignal mindestens zweier zweiter Verarbeitungs-/Verknüpfungsstufen 3.13 und 3.14, welche aus den verarbeiteten Sensorsignalen eine Steuergröße für die Applikationsmittel oder einen klinischer Indikator oder Prädiktor als Ausgangssignal der zweiten Verarbeitungs-/Verknüpfungsstufe erzeugt. Der Unterschied liegt darin, daß die unmittelbaren Steuergrößen (beispielsweise ausbleibender Herzschlag bei einem Schrittmacher) wie bisher unmittelbar gebildet werden. Bei einem klinischen Indikator oder Prädiktor handelt es sich hingegen um einen durch die Sensormittel aufgenommen verdeckten Signalzustand, der zeitversetzt aufgenommen, einer längeren Verarbeitungszeit unterliegt und gegebenenfalls iterativ und/oder im Kontext mit anderen Signalzuständen aufgenommen wird. Für das vorliegende System ist von Bedeutung, daß die Indikator- oder Praediktorsignale mit derselben Systemstruktur verarbeitet werden, wie die unmittelbaren Steuersignale, wodurch sich eine wesentliche Vereinfachung im Steuerungsaufwand ergibt. Die Prädiktorsignale unterscheiden sich von den Indikatorsignalen dadurch, daß sie einen konkreten zukünftigen Erwartungszeitraum für einen künftigen Patientenzustand enthalten, während ein Indikatorsignal einem nicht näher definierten Zeitbereich zugeordnet ist, der mit dessen Festlegung beginnt. Die Verarbeitung der zeitlichen Bedingungen ist weiter unten näher dargestellt.

Das mindestens eine Steuersignal, ein klinischer Indikator oder Prädiktor, bilden das Eingangssignal einer dritten Verarbeitungs-/Verknüpfungsstufe, welche ein Therapieapplikator-Rohsignal als Ausgangssignal der dritten Verarbeitungs-/Verknüpfungsstufe 3.15 erzeugt. In dieser Stufe erfolgt die Umsetzung der Indikation oder Praediktion in Verknüpfung mit einem Steuersignal in ein Signal, welches als Therapieapplikator-Rohsignal die Basis für die Behandlung bildet.

Das Therapieapplikator-Rohsignal als Eingangssignal einer vierten Verarbeitungs-/Verknüpfungsstufe 3.16, welche ein verarbeitetes Therapieapplikatoreingangssignal als Ausgangssignal erzeugt. Diese vierte Verarbeitungs- und/oder Verknüpfungsstufe schließt eine Geometriebewertung der Therapieapplikatormittel des Therapieapplikator-Rohsignals ein, welches eine Anpassung an die geometrische Anordnung der Applikationsmittel umfaßt.

Die Arbeitsweise der einzelnen Blöcke ist weiter unten dargestellt. Die wiedergegebene Anordnung ist symbolisch funktionell, da die reale Verarbeitung zeitsequentiell und iterativ erfolgen kann. Von Bedeutung ist aber die dargestellte funktionale blockweise Zusammenfassung der Verarbeitungsstrukturen.

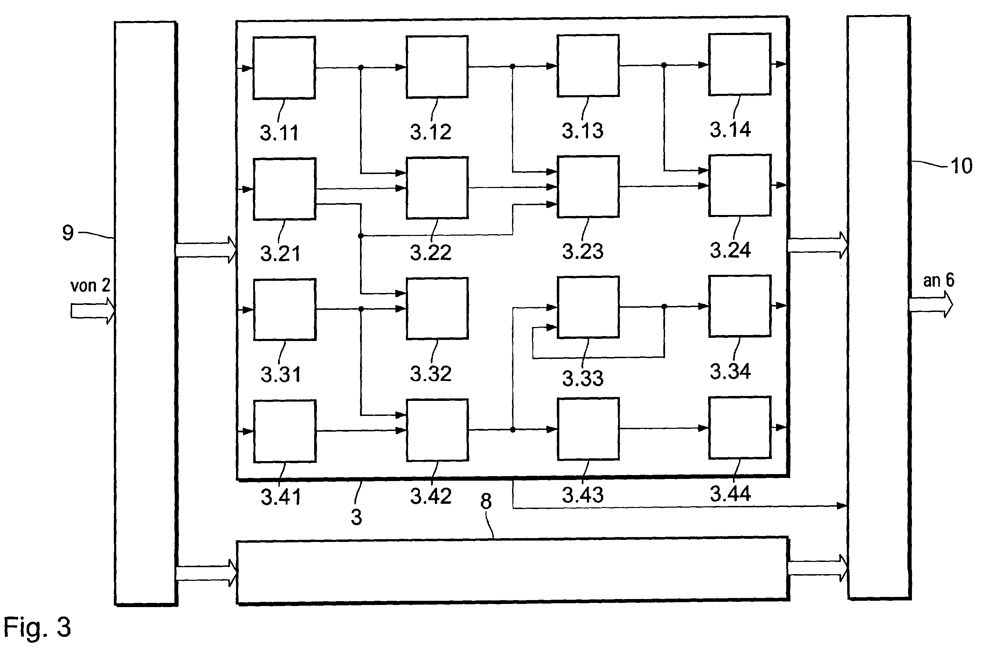

Der in Figur 3 wiedergebende Verarbeitungsteil zeigt einen komplexeren Aufbau als das in Figur 2 wiedergegebene vereinfachte Ausführungsbeispiel. Hierbei ist dargestellt, wie statt einer rein seriellen Verarbeitungsstruktur eine vernetzte Strukur Verwendung finden kann. Es sind jetzt vier Reihen von Verarbeitungs- und Verknüpfungsstufen 3.11 bis 3.14 usw. 3.41 bis 3.44 vorgesehen, welche jeweils vier Baugruppen aufweisen. Jede Baugruppe enthält dabei einen Signalausgang. Die Zahl der Eingänge ist verschieden. Es ist ersichtlich, daß verschiedene Ausgangssignale auch von weiter zurückliegenden Stufen einer nachfolgenden Verarbeitungs- und Verknüpfungsstufe zugeführt werden können (Beispiel: Verarbeitungs-/Verknüpfungsstufe 3.23). Es kann auch das Ausgangssignal einer Stufe (Beispiel: Verarbeitungs-/Verknüpfungsstufe 3.33) zur iterativen Verarbeitung auf sich selbst zurückgeführt werden. Es ist ersichtlich, daß Jede dargestellte Verarbeitungsstufe einen Signal-Daten-Set liefert, welcher eine Information in Form eines Signal-Daten-Sets beinhaltet, die in eine Kernaussage komprimiert ist, welche in nachfolgenden Stufen weiterverarbeitet werden kann. Diese Kernaussage ist mit einer Zeitreferenz für ihre Gültigkeit kombiniert und enthält eine Aussage über einen vorgefundenen Signalzustand, eine Diagnose und eine daraufhin einzuleitende Therapiemaßnahme. Hierbei kann es sich in einfacher Weise um Boolesche Ausdrücke, wie einfache Ja-Nein-Entscheidungen oder Amplitudenwerte handeln. Entsprechend können aber auch Signalverläufe oder Spektren in dem Signal-Daten-Set abgegelegt sein. Es sind jeweils diejenigen Informationen, welche die Basis von weitergehenden Entscheidungen sein können, die zu höherwertigen Aussagen führen. Dabei werden die Informationen entsprechend ihrer zeitlichen Gültigkeit und Ihrer Bezeichnung verknüpft, so daß jederzeit auch Aussagen für ein Verhalten zu anderen Bezugszeitpunkten gewonnen werden können, wenn der entsprechende Zeitbezug gewählt wird.

In Figur 3 ist zusätzlich ein konventionelles System 8 als Backup vorgesehen. Eine Sensordatenverzweigereinheit ist dem Analog-Digital-Wandler 2, der seine Eingangssignale von den Sensormitteln 1 erhält (vgl. Figuren 1 und 2), nachgeschaltet. Die Sensorsignale werden sohls der konventionellem Verarbeitungsteil 8 als auch dem nach dem erfindungsgemäßen Prinzip arbeitenden Verarbeitungsteil 3 zugeführt. Die Verknüpfungs- und Verarbeitungsstufe 3.32 zur Plausibilitätsprüfung gibt bei mangelnder Plausibilität eines Kontroll-Signalzustands im Verarbeitungsteil 3 ein Steuersignal and die Auswahleinheit 10 ab, welche in diesem Fall die Ausgangssignale der konventionellen Verarbeitungsbaugruppe 8 umstellt, die dann über die Digital-Analog-Wandler-Baugruppe 6 die Applikationsmittel 7 ansteuern.

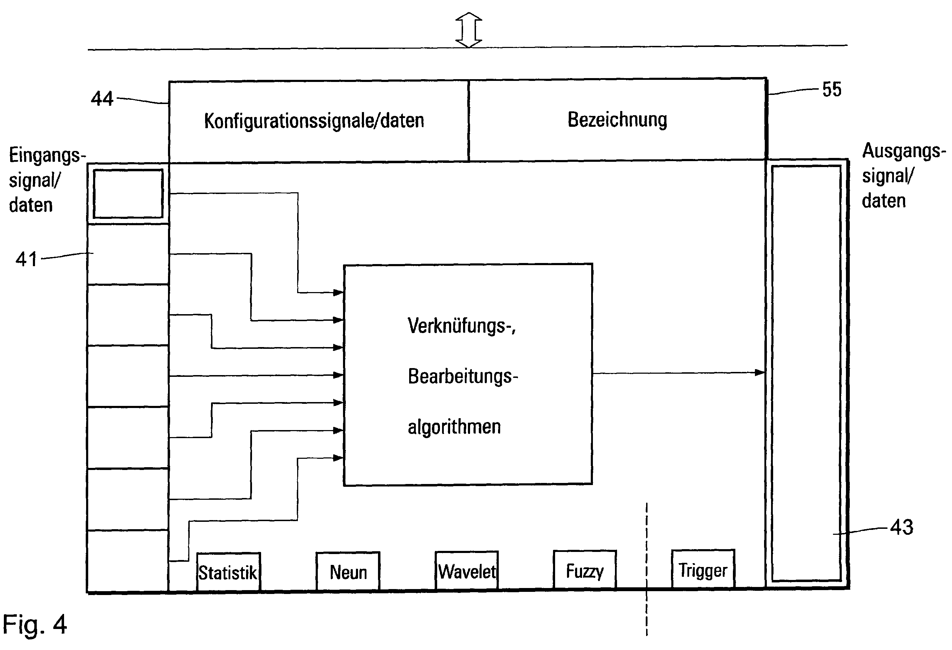

In dem in Figur 4 wiedergegebene Blockschaltbild ist die Struktur einer Verknüpfungs-/Bearbeitungsstufe 3.11 im Detail dargestellt. Diese bildet die physikalische Recheneinheit, welche programmgesteuert die Verarbeitungsoperationen durchführt und dabei auf die im Datenspeicher vorhandenen Größen zurückgreift. Über Eingangsfelder 41 werden in dem Datenspeicher 4 über deren Bezeichnungen und Zeitmarken die durch den in der Speicher für den Verknüpfungs-und Verarbeitungsalgorithmus 42 festgehaltenen Bedingungen selektierten Eingangs-Signal/Signal-Daten-Sets eingelesen. Die zu verarbeiteten Daten werden dann in das Ausgangsdatenfeld 43 überführt und zur Weiterverarbeitung den nachfolgenden Stufen bereitgestellt bzw. in den Datenspeicher 4 zusammen mit der Zeitmarke überführt. Der Verknüpfungs-Verarbeitungsalgorithmus wird bestimmt durch das Konfigurations-Signal-Daten-Set 44, welches ebenfalls im Datenspeicher 4 in Zuordnung zu der Bezeichnung 45 der betreffenden Verknüpfungs- und Bearbeitungsstufe enthalten ist. Die Verarbeitung wird jeweils getriggert durch das Erscheinen oder die Änderung eines Eingangs-Signal-/Daten-Sets in einem Eingangsfeld 41 und wenn alle Zeitreferenzen der Eingangs-Signal-/Daten-Sets gültig sind, d.h. sie die Echtzeit, welche am Anschluß 47 ansteht, als Gültigkeitsbereich enthalten. Eine unbedingte Signalverarbeitung unabhängig von der Echtzeit kann durch eine besondere Triggerbedingung erzwungen über den Zeit-Eingang 47 erzwungen werden, wenn hier eine Zeitangabe übermittelt wird, welche keiner Realen Zeit entspricht. Über derartige Triggerbedingungen können von Extern regelmäßig Service-Operationen erzwungen werden (Beispielsweise in Abhängigkeit von der Systembelastung, wenn beispielsweise im Datenspeicher 4 kein Signal-/Daten-Set vorhanden ist, der eine Zeitreferenz enthält, die kleiner als eine vorgebene Zeitdifferenz vor der Echtzeit gelegen ist.

Für den Verknüpfungs-/Bearbeitungsalgorithmus stehen über die Anschlüsse 46 von extern Plug-Ins zur Verfügung, welche den Verknüpfungs-/Bearbeitungsalgorithmus durch Rechenverfahren unterstützen, welche im Zusammenhang mit der genannten Signalverknüpfungverwendung finden können. Hierbei handelt es sich generell um mathematische Verfahren, welche es erlauben durch entsprechende Informationsverdichtung eine Aussage mit im Kontext hoher Relevanz zu erhalten. Hierzu gehören statistische Verfahrun, Neuro-Verfahren, Wavelet-Verfahren, Fuzzifizierungs-Verfahren sowie Verfahren der statistischen Mechanik.

Das Ausgangs-Signal-/Daten-Set kann also zu dem vorgewählten Zeitpunkt sowohl weitere Datenoperationen ind anderen Verknüpfungs- und Bearbeitungsstufen als auch die Applikatormittel anstoßen.

Die nachfolgende Tabelle enthält die Darstellung des Signal-Daten-Sets, welches bezogen auf einen Bezugszeitpunkt oder einen Bezugszeitbereich verarbeitet wird.

Es ist vergleichbar mit einem Zustandsvektor und wird als Informationseinheit in dem Datenspeicher abgelegt. Das Signal-Daten-Set als Informationseinheit ist individualisiert durch seine Bezeichnung und eine Zeitangabe, welche seine Gültigkeit bestimmt. Dies kann ein Zeitpunkt oder eine Zeitbereich sein, welcher gegebenenfalls für bis auf weiteres gültige Signale nach oben offen ist, also keinen Endzeitpunkt enthält. Alle Signal-Daten-Sets mit gleicher Bezeichnung bilden einen Datensatz, welcher den zeitlichen Verlauf des betreffenden Signal-Daten-Sets charakterisiert. Die Signal-Daten-Sets können Signalspektren, geometrische Formen als Signalamplitudenverläufe, feste Werte, Vektorverweise auf andere Signal-Daten-Sets nach aufgrund von deren Bezeichnung oder Gültigkeitszeitpunkt enthalten. Außerdem kann noch eine Wichtunggröße vorgesehen sein, welche eine Bewertung der statistischen Sicherheit des Signal-Daten-Sets für die Weiterverarbeitung enthält.

Die Signal-/Daten-Sets beschreiben somit sowohl den Patienten- als auch den Systemzustand vollständig, um den Betrieb des Systems zu gewährleisten. Sämtliche Zustandssignale können zur Verarbeitung zusammengeführt, verglichen oder logisch Verknüpft werden, um eine Therapieaktion einzuleiten, welche ebenfalls als Zustandsset festgehalten wird. Die Signal-Daten-Sets bilden sozusagen die das System und den Patienten kennzeichnenden Ereignisse, wobei auch die Zustände zeitvariabel sind und als derartige Ereignisse festgehalten werden.

Ein Signal-Daten-Set ist jeweils einer gleichnamigen Verknüpfungs- und Bearbeitungsstufe zugeordnet. Es beschreibt das an seinem Ausgang vorliegende Signal.

Die Signal-Daten-Sets werden dabei jeweils Zuständen mit der relativ höchsten Relevanz zugeordnet. Dies sind Zustände, welche das Verhalten logisch abhängiger Zustände (im vorliegenden System solcher, die nachgeordneten Verknüpfungs- und Bearbeitungsstufen zugeordnet sind) möglichst eindeutig und ohne Redundanz bestimmen.

Die Signal-Daten-Sets brauchen nicht vollständig alle genannten Signalgrößen enthalten, sondern weisen nur diejenigen auf, welche für die betreffende Verknüpfungs- und Bearbeitungsstufe relevant sind.

Auch die Signale der Sensormittel und die Therapieapplikationsmittel werden durch Signal-Daten-Sets beschrieben.

Entsprechend den Signal-Daten-Sets sind KonfigurationsSignal-Daten-Sets im Datenspeicher festgehalten, welche für die Verknüpfungs- und Bearbeitungsstufen in Zuordnung zu deren Bezeichnungen deren Verknüpfungs- und Verarbeitungsalsogorithmen festhalten. Die Verknüpfungsalgorithmen enthalten die Bezeichnungen der vorangehenden Sensormittel, deren Signale in der jeweiligen Verknüpfungs- und Bearbeitungsstufe verarbeitet werden und den bei der Verknüpfung anzuwendenden Algorithmus. Außerdem wird noch der Verarbeitungsalgorithmus festgehalten. Die Signalspektren und Amplitudenverläufe des Signal-Daten-Sets beschreiben deren Übertragungsverhalten, da in der Systemtheorie Signale und Systemverhalten (beispielsweise in Form der Sprunganwort) mathematisch auf gleichartige Weise beschreibbar sind.

Des weiteren sind noch Geometriesignal-Daten-Sets vorgesehen, welche eine geometrische Korrektur für dioe Signale der Sensormittel und Therapie-Applikationsmittel entsprechend deren räumlicher Verteilung im Körper vorsehen. So bilden die gemeinsamen Signal von Elektroden im Falle der Elektrostimulation beispielsweise insgesamt ein Signalbild, welches für die einzelnen Elektroden in seine entsprechenden Komponenten zu zerlegen ist. Diese Komponentenzerlegung hängt aber von der geometrischen Elektrodenkonfiguration ab, wo die entsprechende Konfiguration durch das GeometrieSignal-Daten-Set erzeugt wird.

Sämtliche Signal-/Daten-Sets im Speicher können nach extern übertragen und umgekehrt von dort eingelesen werden. Auf diese Weise ist eine Kommunikation nach extern möglich und es kann eine entsprechende Verarbeitung stattfinden.

Dasselbe gilt für die Systemkonfigurationsdaten. Die Verknüpfungs- und Bearbeitungsstufen enthalten

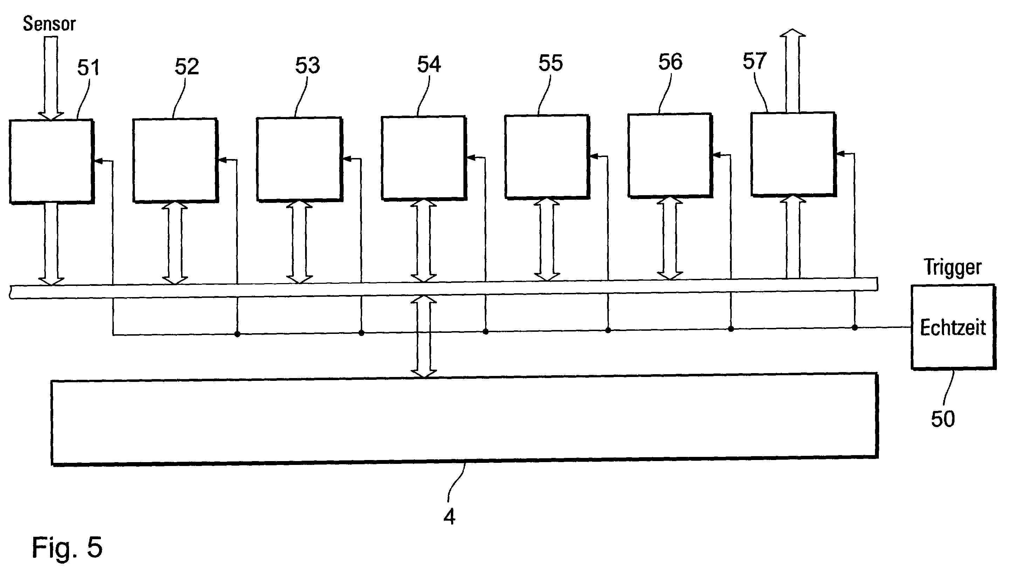

In der Darstellung gemäß Figur 5 ist der Verarbeitungsteil beispielhaft schematisch in mehrere Baugruppen unterteilt, welche funktionsbezogen komplexe Verknüpfungs- und Bearbeitungseinheiten, die ihrerseits in sich komplexer aufgebaut sein können und intern eine ganze Reihe von Verknüpfungs- und Bearbeitungsstufen, wie sie in Figur 4 dargestellt ist, enthalten können. Derartige komplexe aufgebaute Verknüpfungs-/Bearbeitungsmodule können daher im Gegensatz zu einzelnen Verknüpfungs- und Bearbeitungsstufen mehrere Ausgänge enthalten. Dies ist jedoch nicht notwendigerweise der Fall. Sie verhalten sich wie zusammengesetzte Verknüpfungs- und Bearbeitungsstufenm, sind in ihrer Ansteuerung jedoch nicht so variabel und transparent, weil sie festgefügte Funktionen ausführen, welche nicht zugänglich oder veränderbar sein sollen, um die die Betriebssicherheit des Systems nicht durch falsche Manipulationen zu gefährden. So ist es beispielsweise möglich, eine komplette herkömmliche Herzschrittmacherfunktion in ein derartiges komplexes verknüpfungs-/Bearbeitungsmodul einzubinden, wobei dieses System seine eine Anzahl von Signal-/Daten-Sets mit Beichnungen und den betreffenden Referenzzeiten in den Datenspeicher einstellt und dort für das übrige System zur Verfügung stellt. Über "Handles", welche Systemdaten-Sets bilden, können die entsprechenden verknüpfungs-/Bearbeitungsmodule sowohl von Verknüpfungs- und Bearbeitungsstufen als auch von extern angesprochen werden. Die Verknüpfungs-/Bearbeitungsmodule, welche auf diese Weise das Verhalten bekannter Therapiegeräte nachbilden können, sind dann entsprechend diesen programmierbar, wobei die Programmiereingaben über Verknüpfungs- und Bearbeitungsstufen festgelegt werden können. Dies hat den Vorteil, daß den Ärzten geläufige Systeme als solche implementiert werden können und somit den von extern über Telemetriemittel einseh- und beeinflußbaren Aufbau nicht unnötig verkomplizieren. Die Bediener können sich also auf solche Systeme und Eigenschaften konzentrieren, welche der therapeutischen Aufmerksamkeit beispielsweise deswegen offen sein müssen, weil es sich dabei um Maßnahmen oder Eigenschaften handelt, deren Einwirkung auf den Patienten noch nicht in vollem Umfang gesichert ist.

Bei der Darstellung in Figur 5 sind mehrere derartige verknüpfungs-/Bearbeitungsmodule (nachfolgend auch kurz "Modul" genannt) 51 bis 57 dargestellt, welche mit dem Datenspeicher 4 in Wechselwirkung stehen. In der Darstellung sind ein Grund-Modul 51, welches von den Sensormitteln erhaltenen Eingangsdaten erfaßt und beispielsweise die Applikationszeiten für die Grundtherapie ermittelt und weiteren Module 52 bis 57 dargestellt, von denen das Modul 57 die Applikationsmittel ansteuert, wobei hier verschiedene vorgebene Therapiemuster über die abrufbar sind. Der Abruf erfolgt wiederum dadurch, daß die Echtzeit oder eine intern gesetzte Zykluszeit einen vorgegebenen von einem anderen Modul gesetzte Vorgabezeit erreicht, wobei das Erreichen der vorgegebenen Zeit dann - wie weiter oben beschrieben - als "Trigger" wirkt (Block 50).

Die Funktionen der anderen Module 52 bis 56 können verschieden gewählt sein. Als Beispiel wird nachfolgend eine Reihe von komplexen Funktionen dargestellt, die durch Verknüpfungs-/Bearbeitungsmodule ausgeführt werden können:

Verschiedene Zeit oder Ereignisgetriggerte Funktionen, welche die Arbeitsweise des Grundmoduls 51 ergänzen. Dabei kann ein derartiges Modul insbesondere hinsichtlich seines inneren Aufbaus transparent, d.h. in allen seinen Verknüpfungs- und Bearbeitungsstufen (vgl. Figur 3) von außen zugänglilch gestaltet sein.

Verdichtung von Daten im Speicher im Sinne einer Signalkompression. Anstelle der einzelnen in ihrem Amplitudenverlauf oder ihren spektralen Eigenschaften in Zuordnung zu den jeweiligen Zeitreferenzen festgehaltenen Signalen werden die Ereignisse nur noch klassifiziert als Ereigniszahlen für jeweils zugeordnete Zeiträume festgehalten (Histogramme) oder in anderer Form als Signalstatistik festgehalten.

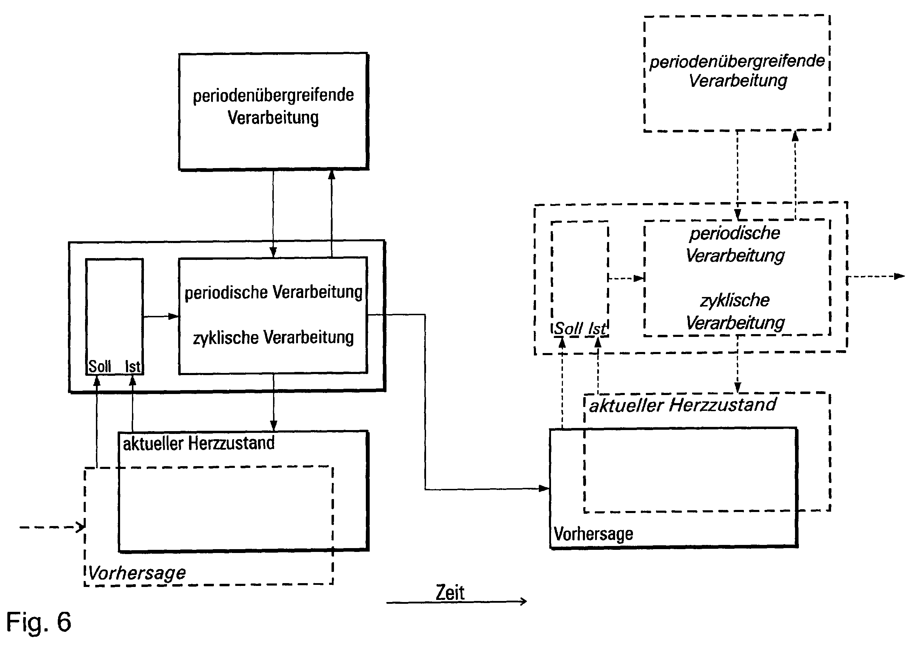

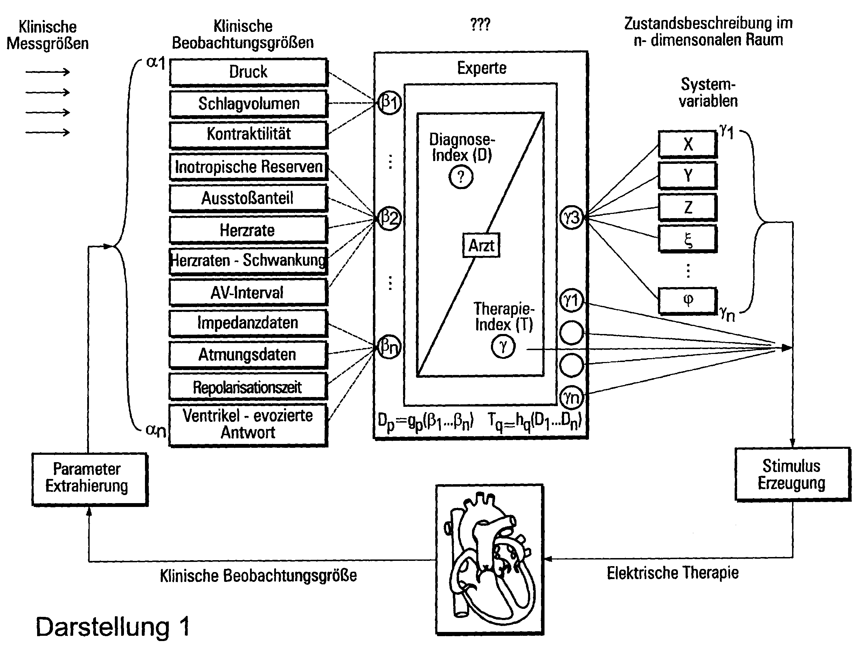

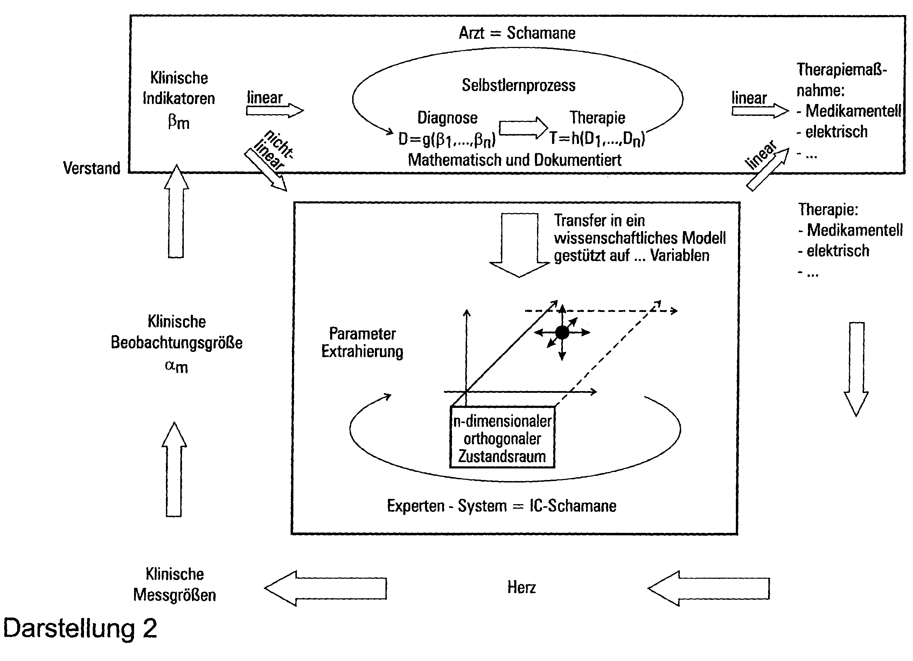

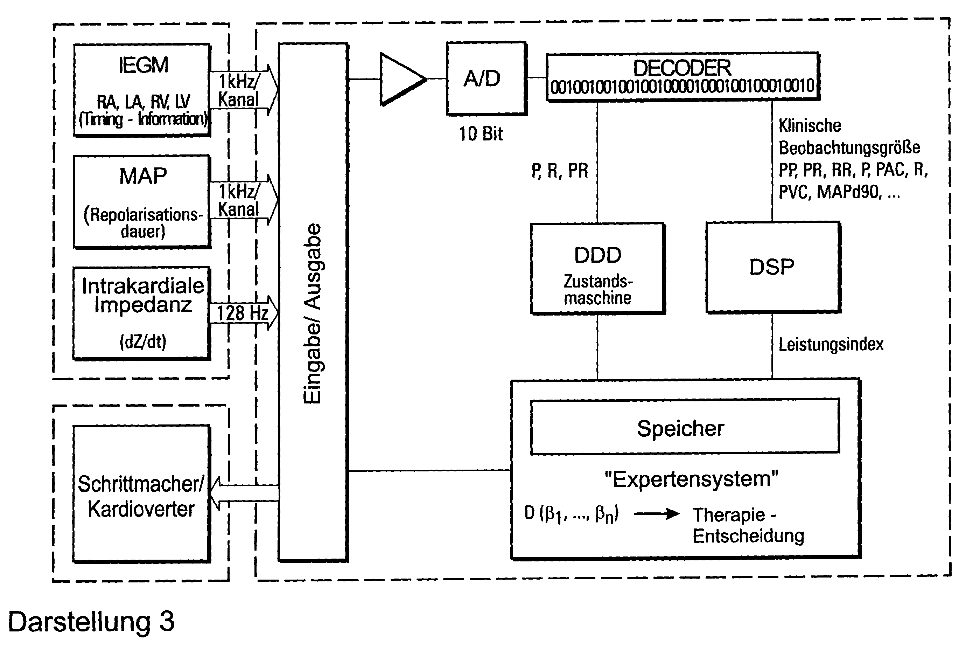

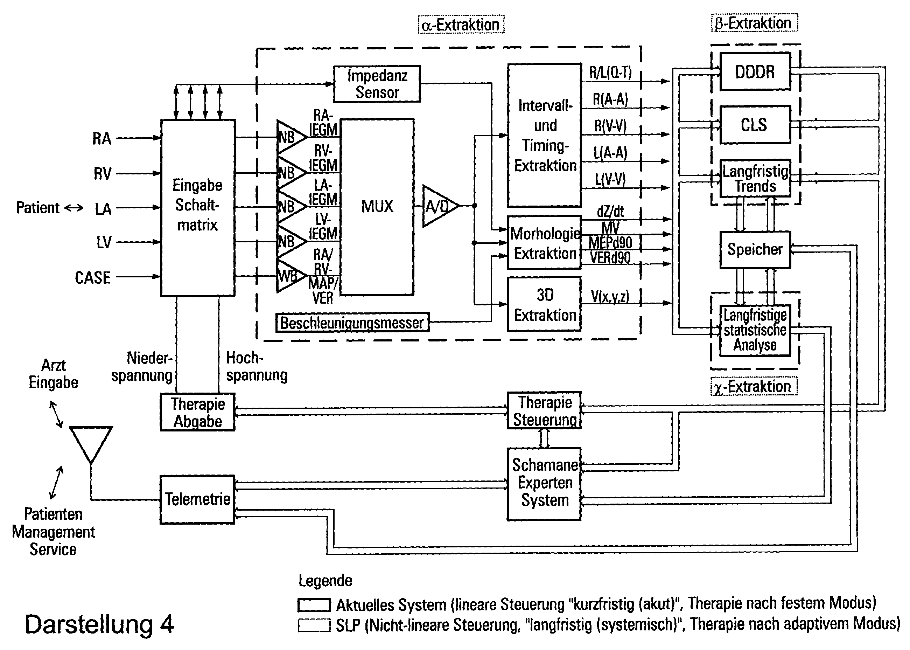

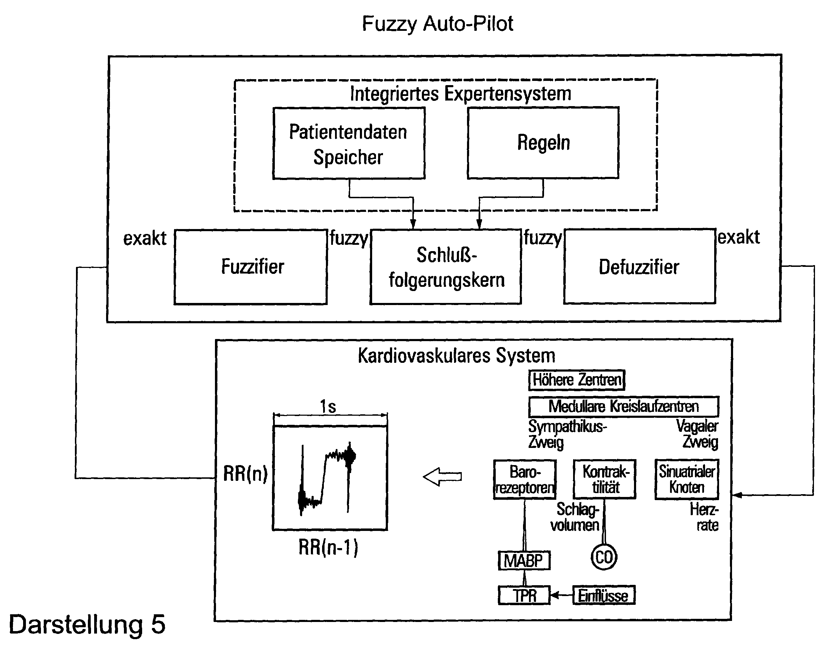

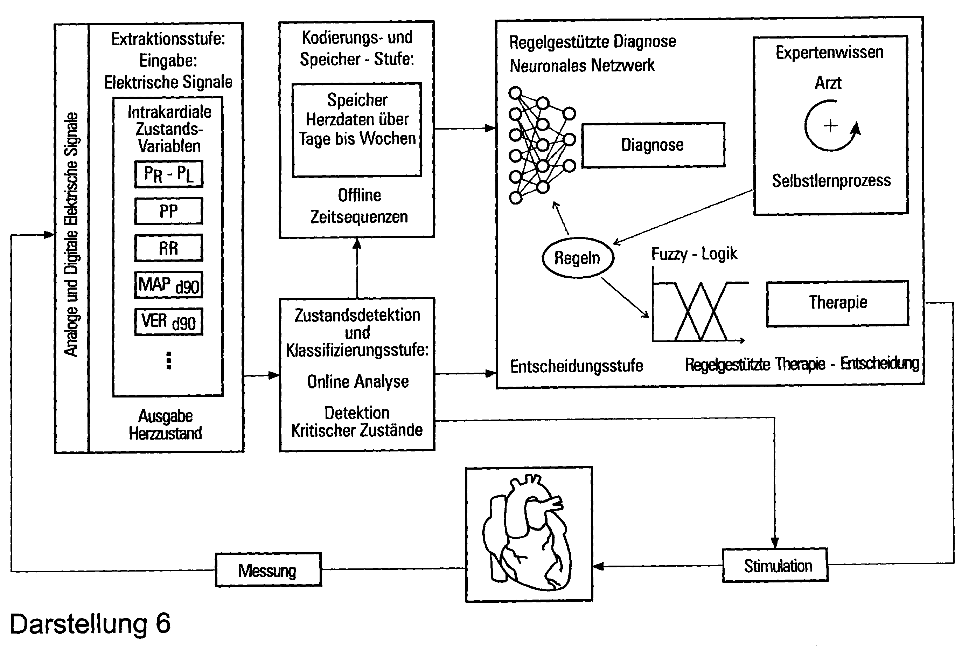

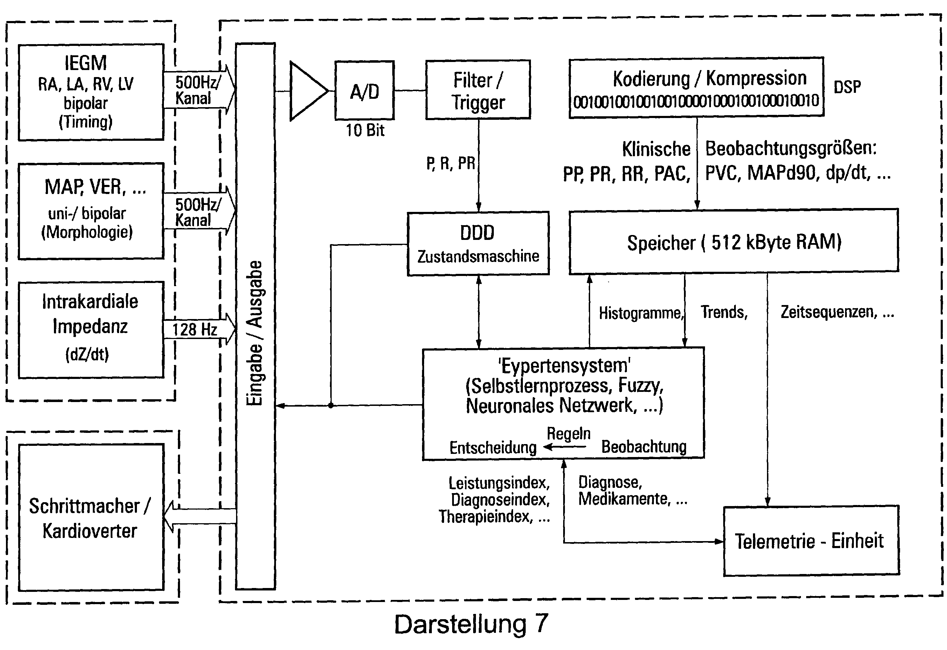

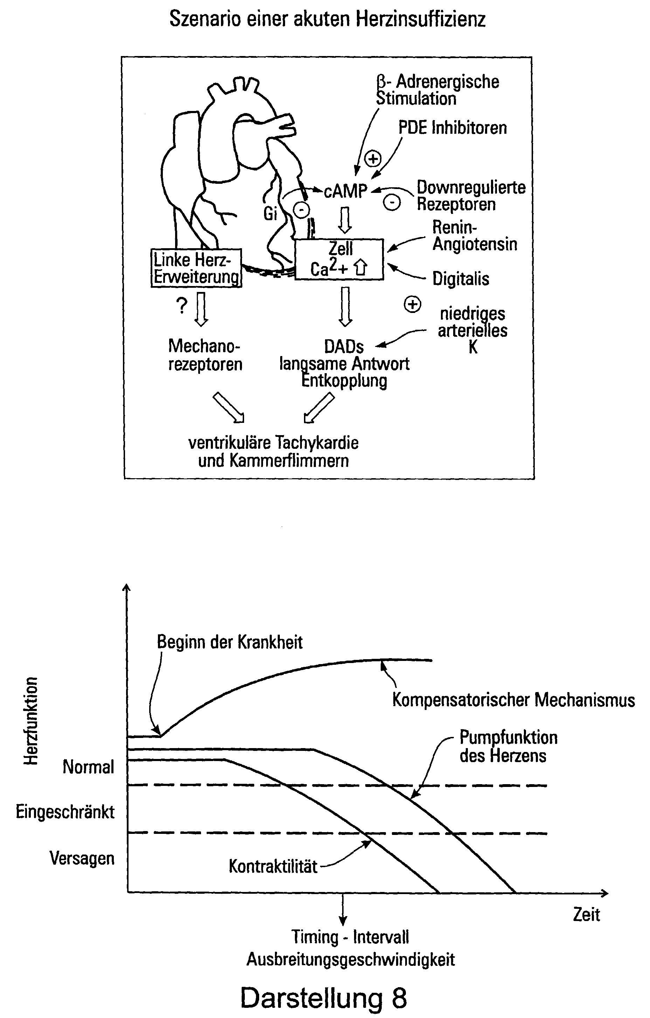

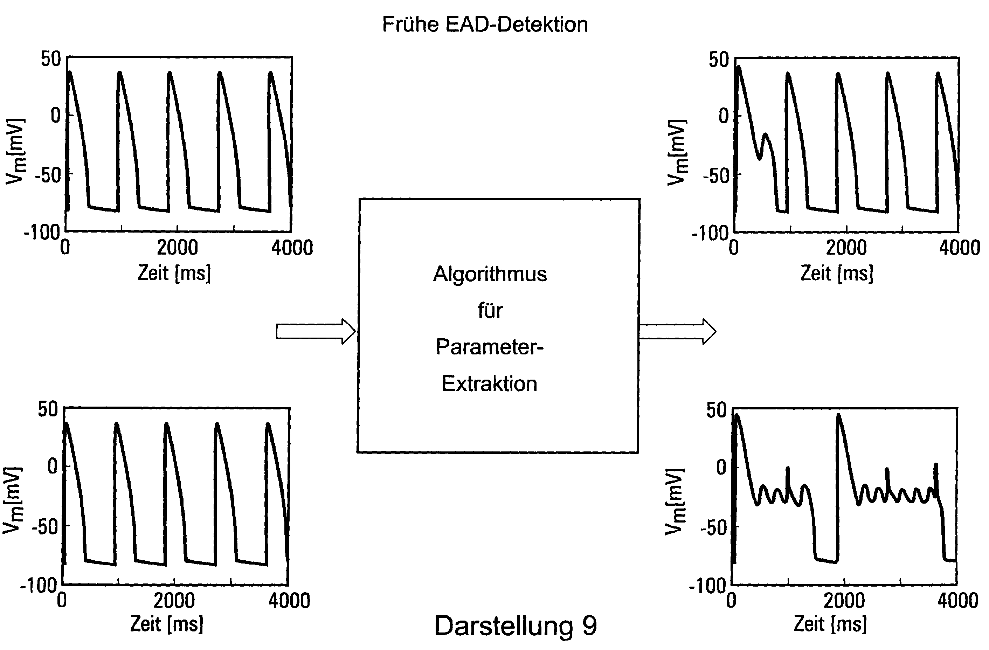

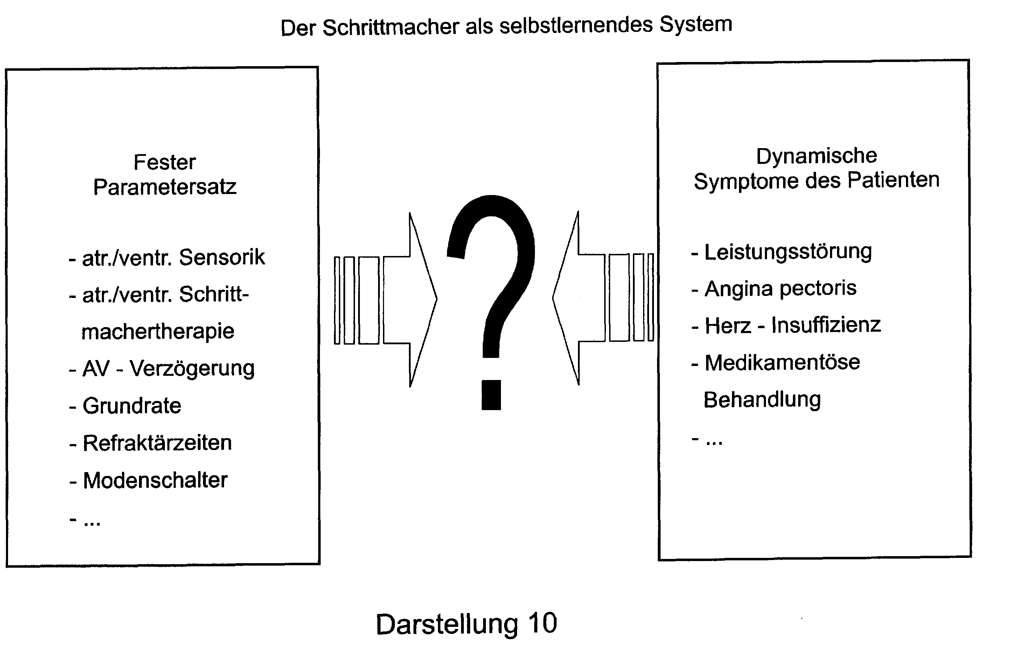

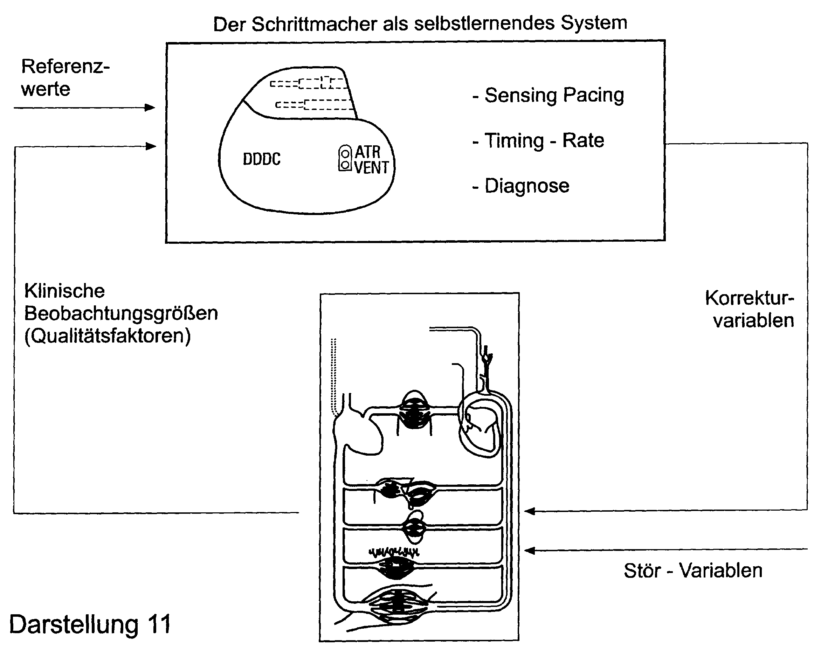

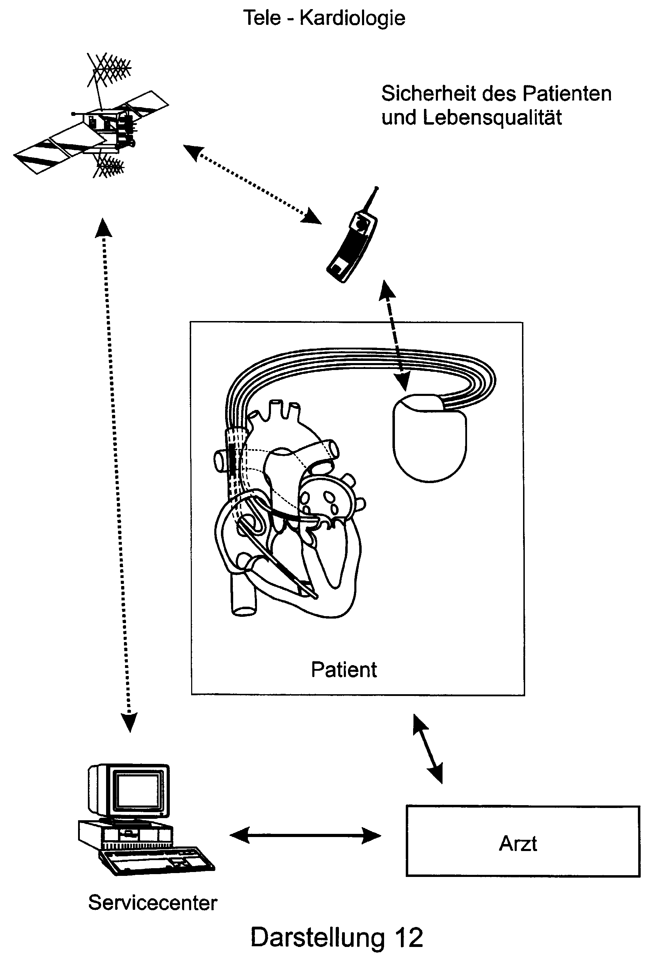

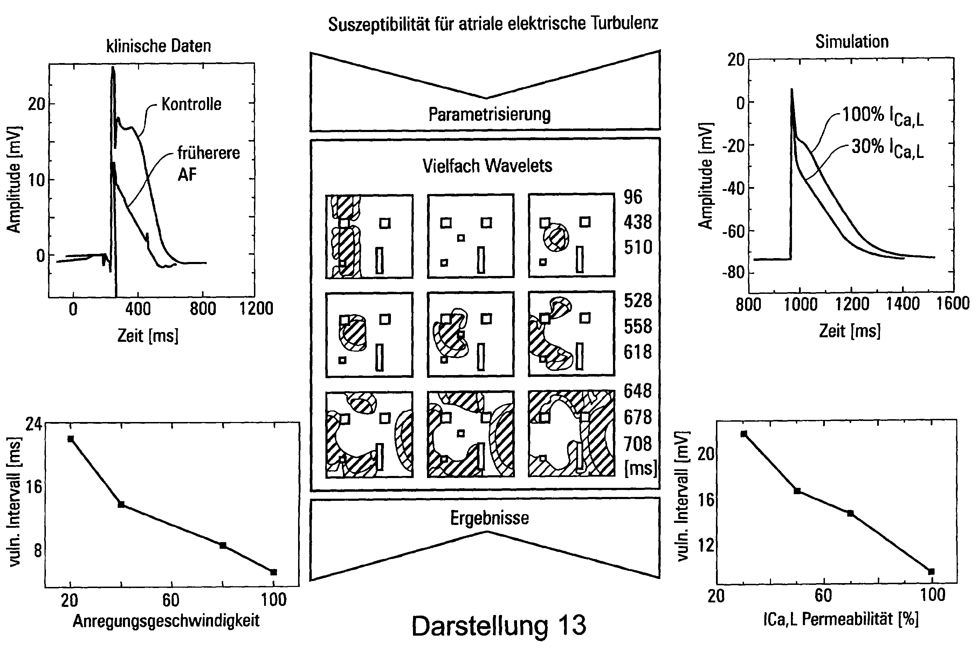

In Figur 6 ist schematisch dargestellt, wie sich bei dem erfindungsgemäßen System die aufgrund der Indikatoren erzeugte Vorhersage mit dem tatsächlich eingetretenen Zustand verglichen und daraus ein Soll-Ist-Vergleich durchgeführt wird.

Die weiteren in der Anlage beigefügten 13 Darstellungen zeigt nach der Erfindung ausgeführte Systeme in ihrer Wirkungsweise oder Teile derselben.
Zu den weiteren Ausführungsmöglichkeiten wird auf die Ansprüche verwiesen. Die Erfindung beschränkt sich in ihrer Ausführung nicht auf die oben erläuterten bevorzugten Ausführungsbeispiele. Vielmehr ist eine Anzahl von Varianten denkbar, welche von der dargestellten Lösung auch bei anders gearteten Ausführungen Gebrauch machen.

## Patentansprüche

1. Medizinisches Therapiesystem, vorzugsweise zur Elektrostimulation oder sonstigen Behandlung des Herzens mit elektrischen Impulsen, mit zwischen eingangsseitigen Sensormitteln und ausgangsseitigen Therapie-Applikatormitteln eingeschalteten Verknüpfungs- und/oder Bearbeitungsstufen für Signale , dadurch gekennzeichnet, daß aus den Eingangsignalen eine Signalgröße durch die Verknüpfungs-/Bearbeitungsstufen und/oder in einem aus mehreren Verknüpfungs-/Bearbeitungsstufe bestehenden Verknüpfungs-/Bearbeitungsmodul aus den Eingangsignalen mindestens ein Indikatorsignal ermittelt wird, das ein Maß für ein wahrscheinlich bevorstehendes therapiepflichtiges Ereignis oder den Therapieerfolg bildet.

2. Therapiesystem, insbesondere nach einem der vorangehenden Ansprüche, dadurch gekennzeichnet, daß das Indikatorsignal zusätzlich ein Zeitreferenzsignal aufweist, welches den wahrscheinlichen Zeitpunkt des Auftretens des therapiepflichtigen Ereignisses oder des Therapieerfolgs beinhaltet (Präediktor).

3. Therapiesystem, insbesondere nach einem der vorangehenden Ansprüche, dadurch gekennzeichnet, daß es sich bei den Eingangssignalen um klinische Observationsgrößen, und dabei im Falle der Herztherapie insbesondere um Arrhythmieindikatoren, handelt.

4. Therapiesystem, insbesondere nach einem der vorangehenden Ansprüche, dadurch gekennzeichnet, daß sich bei den Eingangssignalen zur Ableitung von Indikatorsignalen für Arrhythmien um das Auftreten von Extrasystolen, der Grad der Vorzeitigkeit von Extrasystolen, die Anzahl vorzeitiger Herzkontraktionen, arrhythmische Muster, Leitungszeiten innerhalb der Herzkammern.

5. Therapiesystem, insbesondere nach einem der vorangehenden Ansprüche, dadurch gekennzeichnet, daß das Indikatorsignal zusätzliche eine Wahrscheinlichkeitsbewertungsgröße hinsichtlich der Wahrscheinlichkeit des Auftretens des therapiepflichtigen Ereignisses oder des Therapieerfolgs beinhaltet.

6. Therapiesystem, insbesondere nach einem der vorangehenden Ansprüche, dadurch gekennzeichnet, daß die Extraktion unter mindestens einfacher Anwendung von einem oder mehreren Verfahren erfolgt, welche ein oder mehrstufige mathematische Verfahren oder Filtermittel zum Auffinden eines Signalereignisses in einem Signalgemisch enthalten.

7. Therapiesystem, insbesondere nach Anspruch 4, dadurch gekennzeichnet, daß als mathematische Verfahren oder Filtermittel zum Auffinden eines Signalereignisses in einem Signalgemisch mindestens eines der nachfolgenden vorgesehen ist:
Korrelation, Mustervergleich, Vergleich der Signalsets als Zustandsvektoren insbesondere nach der Methode der kleinsten Fehlerquadrate, Fuzzy-Logik, Wavelets, Nichtlineare Verknüpfungen, Lorenzattraktoren, mathematische Funktionen mit mehreren Variablen, n-dimensionale orthogonale Systeme handelt.

8. Therapiesystem, insbesondere nach einem der vorangehenden Ansprüche, dadurch gekennzeichnet, daß die Extraktion durch die Verknüpfung der Erfahrungswerte von mehreren Therapiesystemen bei gleichartigen Therapiefällen erfolgt.

9. Therapiesystem, insbesondere nach einem der vorangehenden Ansprüche, dadurch gekennzeichnet, daß mindestens ein Sensormittel-Roh-Signal als Eingangssignal einer ersten Verknüpfungs-/Bearbeitungsstufe zugeführt wird, wobei ein verarbeitetes Sensorsignal als Ausgangssignal der ersten Verknüpfungs-/Bearbeitungsstufe erscheint, wobei die erste Verknüpfungs-/Bearbeitungsstufe eine Geometriebewertung der Sensormittel und/oder eine Zeit- und/oder Frequenzkorrektur der Sensorausgangssignale einschließt.

10. Therapiesystem, insbesondere nach einem der vorangehenden Ansprüche, dadurch gekennzeichnet, daß mindestens ein verarbeitetes Sensorsignal als Eingangssignal mindestens einer zweiten Verknüpfungs-/Bearbeitungsstufe zugeführt wird, deren Ausgangssignal das Indikatorsignal bildet, das ein Maß für ein wahrscheinlich bevorstehendes therapiepflichtiges Ereignis oder den Therapieerfolg ist.

11. Therapiesystem, insbesondere nach einem der vorangehenden Ansprüche, dadurch gekennzeichnet, daß das das Indikatorsignal das Eingangssignal einer dritten Verknüpfungs-/Bearbeitungsstufe bildet, welche als Ausgangssignal ein Therapieapplikator-Rohsignal liefert.

12. Therapiesystem, insbesondere nach einem der vorangehenden Ansprüche, dadurch gekennzeichnet, daß das Therapieapplikator-Rohsignal das Eingangssignal einer vierten Verknüpfungs-/Bearbeitungsstufe bildet, welche als Ausgangssignal ein Therapieapplikatorsigal liefert, wobei die vierte Verknüpfungs-/Bearbeitungsstufe insbesondere eine Geometriebewertung der Therapieapplikatormittel und/oder eine Zeit- und/oder Frequenzkorrektur des Therapieapplikator-Rohsignals einschließt.

13. Therapiesystem, insbesondere nach einem der vorangehenden Ansprüche, dadurch gekennzeichnet, daß eine mehrfach verwendbare Verarbeitungs- und Verknüpfungsschaltung oder Softwaremodul vorgesehen ist, welches als erste, zweite dritte und/oder vierte Verknüpfungstufe zeitlich sequentiell einsetzbar ist, wobei dem Verknüpfungsmodul jeweils neben den Eingangssignalen noch folgene Informationen zugeführt werden: die Art der zu verknüpfenden und zu bearbeitenden Eingangssignale, ein Verarbeitungs- und/oder ein Verknüpfungsalgorithmus.

14. Therapiesystem, insbesondere nach einem der vorangehenden Ansprüche, dadurch gekennzeichnet, daß das Verarbeitungs-und Verknüpfungsmodul zusätzlich eine Verknüpfungsinformation d.h. eine Information darüber, welche Eingangsgrößen in welcher Weise zu überlagern sind, aufweist.,

15. Therapiesystem, insbesondere nach einem der vorangehenden Ansprüche, dadurch gekennzeichnet, daß das Verhalten einer Verknüpfungs-/Bearbeitungsstufe durch mittels eines in einen Speicher einlesbaren Systemdatensets veränderbar ist.

16. Therapiesystem, insbesondere nach einem der vorangehenden Ansprüche, dadurch gekennzeichnet, daß mindestens eine der folgenden Signalgruppen: Ausgangssignale von Sensormitteln, Indikatorsignale, Patientenzustandssignale, Therapieapplikationssignalen, Systembeschreibungssignale, Ein- und/oder Ausgangssignale von Verknüpfungs-/Bearbeitungsstufen, Systemkonfigurationsdaten, ein Signal-/Daten-Set bildet, welches eine Referenzzeitangabe und/oder einen Gültigkeitszeitbereichsangabe aufweist, die den Zeitpunkt kennzeichnet, zu dem die betreffenden Signale oder Daten aufgenommen wurden oder zu dem sie gültig sind, wobei der Anfang oder das Ende des Gültigkeitsbereichs nicht festgelegt zu sein braucht, so daß sie bei der nachfolgenden Verarbeitung entsprechend berücksichtigt oder zugeordnet werden können.

17. Therapiesystem, insbesondere nach einem der vorangehenden Ansprüche, dadurch gekennzeichnet, daß die Signal-/Daten-Sets in einem gemeinsamen Speicher vorgesehen sind.

18. Therapiesystem, insbesondere nach einem der vorangehenden Ansprüche, dadurch gekennzeichnet, daß es sich bei der Referenzzeitangabe um eine Echtzeitangabe oder um eine Zeitangabe handelt, welche sich auf eine zyklische Zeitperiode einer Patientenzustandsgröße oder einer Therapiegröße handelt.

19. Therapiesystem, insbesondere nach einem der vorangehenden Ansprüche, dadurch gekennzeichnet, daß dem Signal-/Daten-Set eine alphanumerische Kennzeichnung zugeordnet ist, welche zur Identifikation und zur Auswahl des entsprechenden Signal-/Daten-Sets dient.

20. Therapiesystem, insbesondere nach einem der vorangehenden Ansprüche, dadurch gekennzeichnet, daß das Signal-/Daten-Set mindestens eine der folgenden Informationen enthält: Boolesche Logikdaten, Amplitudendaten, Amplitudenverlaufsdaten, Spektralinformationsdaten, welche insbesondere der Referenzeit zugeordnet ist, Angaben zur geometrischen Anordnung der Sensor- und/oder Applikatormittel, Patientenzustandsdaten, Systemkonfigurationsdaten, Verknüpfungsalgorithmen, Verarbeitungsalgorithmen.

21. Therapiesystem, insbesondere nach einem der vorangehenden Ansprüche, dadurch gekennzeichnet, daß ein Trigger für die Auslösung einer Aktualisierung der Ausgangsignale der Verknüpfungs-/Bearbeitungsstufe eine Veränderung der diese betreffenden Signale oder Daten ist, insbesondere der Eingangssignale, oder der für diese Verknüpfungs-/Bearbeitungsstufe relevanten Signal-/Daten-Sets.

22. Therapiesystem, insbesondere nach einem der vorangehenden Ansprüche, dadurch gekennzeichnet, daß eine Bedingung für die Auslösung einer Aktualisierung der Ausgangsignale der Verknüpfungs-/Bearbeitungsstufe die Erfüllung einer Bedingung für die Referenzzeit- und -zeitbereichsangaben ist.

23. Therapiesystem, insbesondere nach einem der vorangehenden Ansprüche, dadurch gekennzeichnet, daß Verknüpfungs-/Bearbeitungsstufen oder aus mehreren Verknüpfungs-/Bearbeitungsstufe bestehende Verknüpfungs-/Bearbeitungsmodule vorgesehen sind, welche unabhängig oder in Koordination mit den auf Ausgangssignale von Sensormitteln ansprechenden Verknüpfungs-/Bearbeitungsstufen mindestens eine der nachfolgenden Operationen, insbesondere durch Datenübertragung über Telemetriemittel zu einer entfernt gelegenen Datenverarbeitungsstation und/oder in der Reihenfolge der Dringlichkeit, insbesondere in der Reihenfolge der den Signal-/Daten-Sets zugeordneten Zeitreferenzen, ausführen:
Kompression des für die Signal-/Daten-Sets benötigten Speicherbedarfs durch Reduzierung der den Signal-/Daten-Sets zugeordeneten Signale und/oder Daten durch Ersetzen von komplexeren Datenstrukturen durch einfachere mit zunehmenden Alter der den Signalen/Daten zugeordneten Referenzzeiten, insbesondere Vorsehen einer zusätzlichen Verarbeitungs- und Verknüpfungsstufe zum Verdichten von Patientenzustandsdaten indem mehrere Signal-Daten-Sets von verschiedenen Zeitpunkten oder Zeiträumen zugeordneten Patientenzustandsdaten zu einem größeren Zeitraum zugeordneten Patientenzustandsdaten zusammengefaßt werden. (Signalverdichtung), wobei diese Verdichtung um so größer ist, je weiter die Zeitpunkte oder Zeiträume gegenüber der aktuellen Systemzeit zurückliegen.
Prüfung der Signal-/Daten-Sets auf mögliche Inkonsistenzen, Korrektur inkonsistenter Daten/Signale bzw. Löschung derselben.
Durchführung zu Untersuchungen hinsichtlich der dynamischen Stabilität, des Systems bei möglichen ungewöhnlichen Daten /Signal-Zuständen.
Durchführung von Untersuchungen hinsichtlich Funktionsfähigkeit des Systems und insbesondere Umschaltung der Erzeugung der Therapiesignale durch ein zusätzlich vorgesehens Reserve- und/oder konventionelles System bei einer verbleibenden Abweichung zwischen vorhergesagtem um eingetretenen Therapieerfolg und Stillsetzen des vorher aktiven Systems.
Vergleich des zu einem Indikator (Präediktor) für die Vorhersage des wahrscheinlich bevorstehendes therapiepflichtigen Ereignisses oder den Therapieerfolg gehörigen Signal-/Daten-Sets oder Teile desselben mit dem zu dem tatsächlich eingetretene therapiepflichtigen Ereignis oder dem Therapieerfolg ses oder ergesagten mit dem eingetretenen Therapieerfolg gehörigen Signal-/Daten-Set oder Teilen desselben und bei Überschreiten einer vorgegebenen Abweichung Änderung mindestens eines vorgegebenen Systemzustandsparameters um einen vorgegebenen Betrag.
Umrechnen vom Frequenzspektren in zeitabhängige Signale und umgekehrt (Fast-Fourier bzw. Wavelet-Transformation) eines Signal-Daten-Sets.

24. Therapiesystem, insbesondere nach einem der vorangehenden Ansprüche, dadurch gekennzeichnet, daß die Verknüpfungs- und/oder Bearbeitungsalgorithmen durch sogenannte Plug-Ins oder Applets als zusätzliche Prozessor- oder Softwareteile gebildet werden, welche, sich in einem gemeinsamen Speicher befinden und/oder nach Bedarf über Telemetriemittel aus einer äußeren Quelle geladen werden.

25. Therapiesystem, insbesondere nach einem der vorangehenden Ansprüche, dadurch gekennzeichnet, daß die externe Verarbeitung und/oder die Berechnung eines Indikators unter Berücksichtigung und/oder Zugrundelegung der Signale/Daten eines Kollektivs gleichartiger Systeme mit vergleichbaren Signal/Dateninhalten und/oder einem datenmäßig nachgebildeten Patientenmodell erfolgt.

26. Therapiesystem, insbesondere nach einem der vorangehenden Ansprüche, dadurch gekennzeichnet, daß die Telemetrie die Benutzung des Internet einschließt

27. Therapiesystem, insbesondere nach einem der vorangehenden Ansprüche, dadurch gekennzeichnet, daß die Eingabe von Signalen und/oder Datenwerten des Signal-/Daten-Sets oder Ausführung von Berechnungen von extern über Telemetrie vorgesehen ist.

28. Therapiesystem, insbesondere nach einem der vorangehenden Ansprüche, dadurch gekennzeichnet, daß die Telemetrie mindestens teilweise über das Internet vorgesehen ist.

29. Therapiesystem, insbesondere nach einem der vorangehenden Ansprüche, dadurch gekennzeichnet, daß eine vorausschauende Bearbeitung zur Behandlung von möglicherweise auftretenden Signal-/Datenzuständen vorsorglich erfolgt, wobei ein Abbruch der Verarbeitung erfolgt und/oder die erhaltenen Daten-/Signale verworfen werden, wenn die Voraussetzungen unter denen die vorausschauende Bearbeitung erfolgte, nicht mehr vorliegt.
